# FASCICULE DE BREVET EUROPEEN

(11) **EP 0 390 634 B1**
(45) Date de publication et mention de la délivrance du brevet: **17.05.1995**
(21) Numéro de dépôt: 90400705.1
(22) Date de dépôt: 16.03.1990
(51) Int. Cl.: C08K 9/10, C08L 67/06, G01N 33/543

(54) **Microspheres composites magnetisables de polymere reticule hydrophobe, leur procédé de préparation et leur application en biologie**
Magnetisierbare Mikrokugeln aus vernetztem, hydrophobem Polymer, Verfahren zu ihrer Herstellung und ihre Verwendung in der Biologie
Hydrophobic, cross-linked, polymeric, magnetisable composite microspheres, method for their preparation and their biological uses

(30) Priorité: 31.03.1989 FR 8904231
(43) Date de publication de la demande: 03.10.1990
(73) Titulaire: SOCIETE PROLABO, 94120 Fontenay-sous-Bois (FR)
(72) Inventeur: Charmot, Dominique, F-75019 Paris (FR); Vidil, Christine, F-75018 Paris (FR)
(74) Mandataire: Ahner, Francis

(56) Documents cités:
- EP-A- 0 156 537
- DE-A- 1 959 649
- US-A- 4 339 337

## Description

La présente invention a pour objet des microsphères composites magnétisables de polymères vinylaromatiques réticulés hydrophobes, telles quelles ou en dispersion aqueuse, leur procédé de préparation et leur application en biologie notamment.

Il est connu de préparer des perles magnétisables (brevet US n° 4.339.337) de diamètre allant de 0,05 à 3 mm par polymérisation en suspension d'un monomère vinylaromatique en présence d'un amorceur organosoluble, d'un agent de suspension et d'une charge magnétisable dispersée au sein d'une solution dans ledit monomère d'un polymère non hydrosoluble. Les perles obtenues renferment des charges magnétisables réparties dans la matrice de polymère.

Il a egalement été proposé (brevet US n° 4.358.388) de préparer des latex de polymères hydrophobes magnétisables par homogénéisation d'une solution aqueuse d'émulsifiant et d'une dispersion d'une charge magnétisable dans une phase organique formée d'un amorceur organosoluble , de tout ou partie du monomère hydrophobe et/ou d'un composé organique insoluble dans l'eau, puis polymérisation. Les latex obtenus sont formés de particules de polymère de diamètre d'environ 0,03 à 5 »m renfermant des charges magnétisables réparties dans la matrice polymère, lesdites charges ayant tendance à migrer en périphérie.

La demanderesse a maintenant trouvé des microsphères composites formées d'un coeur (core) essentiellement constitué de particules magnétisables et d'une écorce (shell) de polymère hydrophobe.

Selon l'invention les microsphères composites magnétisables de polymère vinylaromatique réticulé hydrophobe sont caractérisées en ce que :
- elles présentent un diamètre de l'ordre de 0,05 à 10 »m, de préférence de l'ordre de 0,1 à 5 »m ;
- elles comprennent :
   . un coeur essentiellement constitué de particules magnétisables présentant un diamètre inférieur à 300 10⁻⁴ »m et de préférence de l'ordre de 50 10⁻⁴ à 120 10⁻⁴ »m ;
   . et une écorce constituée d'un copolymère réticulé hydrophobe dérivé d'au moins un monomètre vinylaromatique hydrophobe et d'au moins un polymère émulsifiant polyéthyléniquement insaturé soluble dans ledit ou lesdits monomère(s) vinylaromatique(s) et susceptible de réticuler avec ledit ou lesdits monomère(s) vinylaromatique(s).
Parmi les matériaux pouvant constituer les particules magnétisables formant le coeur des microsphères, on peut citer la magnétite, l'hématite, le dioxyde de chrome, les ferrites telles que les ferrites de manganèse, nickel, manganèse-zinc ..., les alliages de cobalt, nickel, gadolinium, samarium-cobalt ... Les matériaux préférentiels sont la magnétite et la l'hématite.

La quantité de particules magnétisables formant le coeur correspond à environ 0,5 à 70 %, de préférence à environ 15 à 60 % du poids de la microsphère composite magnétisable.

Parmi les monomères vinylaromatiques on peut citer ceux du type styrène, alpha-méthylstyrène, éthylstyrène, tertiobutylstyrène, vinyltoluène.

Ces monomères vinylaromatiques sont présents seuls ou en mélange entre eux en toute proportion, ou encore en mélange avec un autre monomère copolymérisable, insoluble dans l'eau, pouvant constituer jusqu'à 50 % du mélange et choisi parmi les composés diéniques, tels que butadiène, isoprène ; les acrylates et méthacrylates d'alkyle dont le groupe alkyle possède 3 à 10 atomes de carbone ; les esters d'acides éthyléniques possédant 4 ou 5 atomes de carbone et d'alkyle possédant 1 à 8 atomes de carbone, tels que : fumarate d'heptyle, fumarate d'octyle, itaconate de méthyle, itaconate d'éthyle.

Peuvent également être présents de 0 à 5 % en poids par rapport au(x) monomère(s), d'un monomère réticulant comme par exemple le divinylbenzène, le méthacrylate de vinyle, les acrylates ou méthacrylates de mono-ou polyalkylène (2-4C) glycol, le cyanurate de triallyle, les condensats d'acides carboxyliques insaturés et de polyols, comme par exemple les acrylate et methacrylate de triméthylol-propane.

Parmi les polymères émulsifiants réticulant avec le ou les monomères vinylaromatiques on peut citer les polyesters d'alkylène glycol et de diacide insaturé aliphatique et/ou aromatique, les polyméres carboxylés de diolefines ... de masse molaire en poids de l'ordre de 500 à 10 000, de préférence de l'ordre de 1 000 à 5 000. Des exemples sont les polyesters de propylène glycol et d'anhydride maléïque, les polyesters de propylène glycol, d'anhydride maléïque et d'anhydride phtalique.

Les polymères émulsifiants réticulants représentent environ de 3 à 15 %, de préférence de 5 à 10 % du poids de monomère vinylaromatique hydrophobe. Ici le terme "monomère vinylaromatique hydrophobe" signifie l'ensemble formé par les monomères vinylaromatiques hydrophobes, leurs comonoméres et leurs comonomères réticulants éventuels.

Les microsphéres composites magnétisables faisant l'objet de l'invention peuvent se présenter telles quelles ou en dispersion dans l'eau ; L'invention a donc encore pour objet une dispersion aqueuse contenant 10 à 70%, de préférence 15 à 50% en poids de microsphères composites magnétisables selon l'invention.

La présente invention a également pour objet un procédé de préparation desdites microsphéres composites magnétisables.

Le procédé consiste :
- à disperser des particules magnétisables présentant un diamètre inférieur à 300 10⁻⁴ »m, de préférence de l'ordre de 80 10⁻⁴ à 120 10⁻⁴ »m, revêtues d'une couche monomoléculaire d'un agent dispersant non hydrosoluble dans un mélange constitué d'au moins un monomère vinylaromatique hydrophobe, d'au moins un polymère émulsifiant polyéthylèniquement insaturé soluble dans ledit ou lesdits monomère(s) vinylaromatique(s) et susceptible de réticuler avec ledit ou lesdits monomère(s) vinylaromatique(s) et éventuellement d'un agent dispersant complémentaire.
- à préémulsionner le fluide magnétique ainsi obtenu dans de l'eau, après introduction préalable dans ledit fluide d'un amorceur organosoluble de polymérisation ;
- à copolymériser les composés éthylèniquement insaturés présents dans le milieu
- à séparer les microsphères magnétisables formées des microsphéres non magnétisables
- et éventuellement à redisperser dans l'eau les microsphères magnétisables.

On entendra ci-après par :
- "monomères vinylaromatiques" l'ensemble formé par les monomères vinylaromatiques, leurs comonomères et les comonomères éventuels réticulants. La nature et leurs quantités respectives ont déjà été mentionnées ci-dessus ; les éventuels comonomères réticulants peuvent être mis en oeuvre soit à l'étape de dispersion, soit à l'étape de préémulsion (ils peuvent alors servir de solvant à l'amorceur de polymérisation).
- "composés éthyléniquement insaturés copolymérisables" l'ensemble formé par les "monomères vinylaromatiques" et le polymère émulsifiant réticulant.

La nature des particules magnétisables a déjà été mentionnée ci-dessus.

Parmi les agents dispersants formant un revêtement mono-moléculaire non hydrosoluble autour des particules magnétisables on peut citer ceux présentant une longue chaîne hydrocarbonée terminée par un groupe polaire du type - COOH, NH₂ ..., tels que les acides gras, les amines grasses ... contenant au moins 12 atomes de carbone et plus particulièrement les acides gras en C₁₈, tels que les acides oléïque, linoléïque et linolénique.

Les particules magnétisables revêtues d'agent dispersant peuvent être préparées par exemple par peptisation dans l'agent dispersant de particules magnétiques obtenues par voie sol-gel, dispersion dans un liquide vecteur organique (brevet US n° 3.843.540) puis floculation à l'aide d'un solvant polaire du type cétone, ester, alcool et séparation des particules enrobées.

Les proportions de particules magnétisables représentent de 0,5 à 60 %, de préférence de 15 à 50 % du poids de composés éthyléniquement insaturés copolymérisables.

L'étape de dispersion des particules magnétisables est réalisée par introduction progressive, sous agitation à une température de l'ordre de 0 à 30°C, des particules magnétisables revêtues d'agent dispersant non hydrosoluble dans les composés éthyléniquement insaturés copolymérisables. Si nécessaire cette étape peut être réalisée en présence d'un agent dispersant complémentaire anionique ou cationique ; à titre d'exemple on peut citer les esters phosphoriques, alkylaromatiques éthoxylés ... ; ledit agent peut être mis en oeuvre à raison de 3 à 15 % du poids des composés éthyléniquement insaturés copolymérisables.

Un amorceur organosoluble de polymérisation est ensuite introduit dans la dispersion, éventuellement sous forme de solution dans un peu de monomère vinylaromatique ou d'un de ses comonomères réticulants ou non.

Il est mis en oeuvre en quantité comprise entre environ 0,1 à 10 % en poids par rapport aux composés éthyléniquement insaturés copolymérisables.

Comme exemple d'amorceur on peut citer les azonitriles comme l'azobisisobutyronitrile, l'azobiscyclohexane carbonitrile ; ou les peroxydes, comme les peroxydes de benzoyle, dicumyle, ditertiobutyle, diacétyle, dioctanoyle, lauroyle, méthyléthylcétone, caprylyle, dichloro-2,4-benzoyle, parachlorobenzoyle ; les perpivalate, diétyl-peracétate, perbenzoate de tertiobutyle ; le diperphtalate de ditertiobutyle ; le 1,1-ditertiobutyle-peroxyde-3,3,5-triméthyl-cyclohexane.

L'étape de préémulsion est réalisée par homogénéisation du fluide magnétique obtenu avec de l'eau, à une température inférieure à celle de décomposition de l'amorceur, à l'aide d'un système d'agitation énergique tel que moulin à colloïdes, pompe à haute pression, agitateur à vibrations, appareil à ultra-sons ... jusqu'à obtenir une émulsion de gouttelettes de fluide magnétique de taille de l'ordre de 0,03 à 10 »m, de préférence de l'ordre de 0,1 à 5 »m.

Cette opération de préémulsion est de préférence réalisée à un pH de l'ordre de 7 à 11.

La quantité d'eau présente est telle que la dispersion aqueuse de microsphères obtenue après copolymérisation contienne de 10 à 65 %, de préférence de 10 à 30 % de son poids desdites microsphères.

Si nécessaire un émulsifiant complémentaire peut être présent ; il peut être anionique, cationique ou non-ionique et utilisé à raison de 0,1 à 5 % en poids par rapport à celui du ferrofluide à préémulsionner.

Parmi les agents émulsifiants anioniques peuvent être cités les sels d'acides gras ; les alkylsulfates, alkylsulfonates, alkylarylsulfonates, alkylsulfosuccinates, alkylphosphates alcalins ; les sulfosuccinates d'alkyle ; les sulfonates d'éthers alkylphénolpolyglycoliques ; les sels d'esters d'acides alkylsulfopolycarboxyliques ; les produits de condensation des acides gras avec les acides oxy-et amino-alcanesulfoniques ; les dérivés sulfatés des éthers polyglycoliques ; les esters sulfatés d'acides gras et de polyglycols ; les alcanolamides d'acides gras sulfatés.

Parmi les agents émulsifiants cationiques peuvent être cités les alkylamines et leurs sels solubles dans l'eau, les sels solubles des alkylamines N-substituées par des radicaux alkyle et/ou alkylaryle et/ou hydroxyalkyle. Et, parmi les agents émulsifiants non ioniques, les esters gras de polyalcools, les alcanolamides d'acides gras, les polyoxydes d'éthylène, les copolyoxydes d'éthylène et de propylène, les alkylphénols oxyéthylénés.

L'opération de polymérisation est réalisée à une température de l'ordre de 30 à 130°C, et de préférence de l'ordre de 50 à 85°C. Elle dure environ de 2 à 10 heures et généralement de 2 à 5 heures.

On obtient ainsi une dispersion aqueuse d'un mélange de microsphères composites magnétisables et de microsphères ne renfermant pas de charges magnétisables (microsphères "blanches"), dont le diamètre est de l'ordre de 0,05 à 10 »m, et généralement de l'ordre de 0,1 à 5 »m. Le taux de microsphères magnétisables peut représenter de 20 à 90 % en poids de la quantité totale de microsphères. Plus la concentration de charges magnétisables dans les microsphères est importante, plus grande est la quantité de microsphères "blanches".

La séparation des microsphères magnétisables est de préférence réalisée par aimantation.

Si désiré, les microsphères magnétisables séparées peuvent être redispersées dans l'eau afin d'obtenir une dispersion renfermant de 10 à 70 % en poids, de préférence 15 à 50 % en poids de microsphères composites magnétisables.

Les microsphères composites magnétisables, telles quelles ou en dispersion aqueuse, ci-dessus décrites peuvent être utilisées en biologie pour immobiliser des substances biologiquement actives (protéines telles que anticorps, enzymes ... ; antigènes ; médicaments ..) par adsorption ou covalence ; selon la nature de ladite substance active les produits de l'invention peuvent être avantageusement utilisés dans les tests de diagnostic (agglutination - "RIA" radioimmunological assay - "IRMA" immunoradiometric assay - "EIA" enzyme immuno assay), en chromatographie d'affinité, comme catalyseur enzymatique en biotechnologie, comme support de culture cellulaire.

Les exemples suivants sont donnés à titre indicatif et ne peuvent être considérés comme une limite du domaine et de l'esprit de l'invention.

### Exemple 1

### 1ère étape : dispersion du pigment magnétique

On introduit :
. 8 g de Résine A 123 (polycondensats de propylène glycol et d'anhydrides maléïque et phtalique, commercialisé par ORKEM).
. et 4 g de GAFAC RE 610 (mélange de mono et diesters phosphoriques de dérivés alkyl-aryl ethoxylés, commercialisé par GAF)
dans 75,5 g de styrène.
On introduit petit à petit sous agitation dans le mélange obtenu 12,5 g de pigment magnétique constitué de 10 g de Fe₃O₄ de diamètre moyen de l'ordre de 80 10⁻⁴ à 120 10⁻⁴ »m surfacté par 2,5 g d'acide oléïque.

### 2ème étape : préémulsion

On ajoute à 40 g du fluide magnétique obtenu, une solution de 0,6 g de peroxyde de dioctanoyle dans 1,5 g de divinylbenzène.

La phase organique obtenue est introduite dans 230 g d'eau amenée à pH 10 par addition de potasse 1 N et dispersée dans la phase aqueuse à l'aide de l'homogénéiseur ULTRA-TURRAX (moteur d'entrainement T 45, tige de dispersion 45 N, générateur T 45/66, commercialisé par PROLABO).

Le diamètre moyen des gouttelettes obtenues est de l'ordre de 0,4 »m (mesure à l'aide du granulomètre laser CILAS 850, commercialisé par CILAS).

### 3ème étape : copolymérisation

La préémulsion obtenue est polymérisée dans un réacteur de 500 l à 80°C pendant 4 heures sous azote. Le milieu est ensuite refroidi ; en cours de refroidissement 50 ml d'une solution aqueuse à 0,5 % en poids de laurylsulfate de sodium sont ajoutés.

Le latex obtenu est filtré sur une toile de 5 »m ; les monomères résiduels sont éliminés par stripping à l'aide d'un évaporateur rotatif.

Les microsphères magnétisables sont ensuite séparées à l'aide d'un aimant ; leur taux représente environ 65 % du poids total de microsphères.

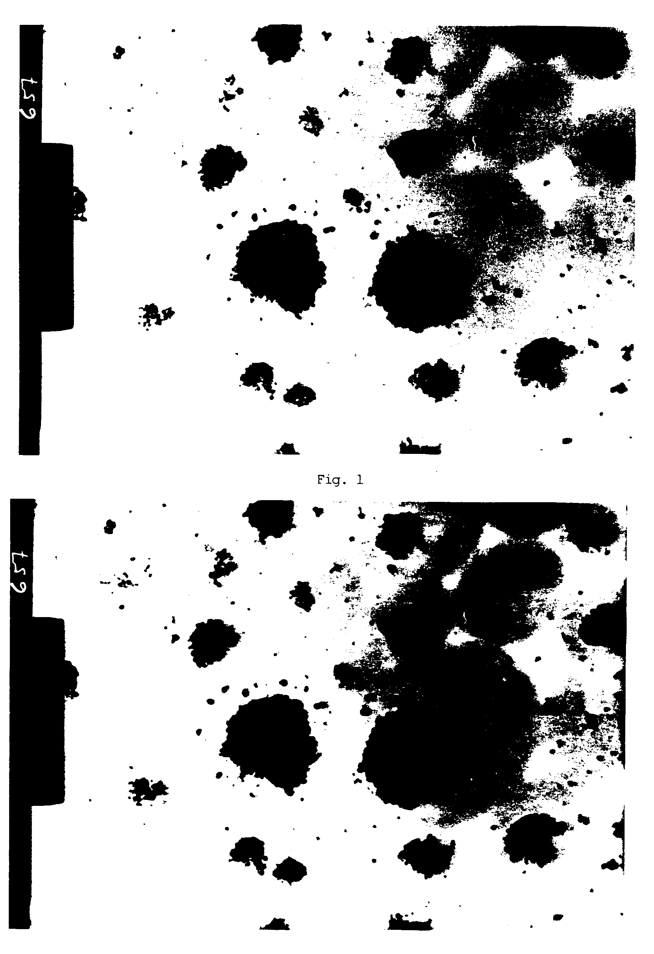

Les microsphères magnétisables présentent un diamètre moyen de l'ordre de 0,4 »m et renferment 18 % de leur poids de particules de ferrite formant le coeur des microsphères, comme le montre la photo de la figure n° 1. (coupe des microsphères vue au microscope à transmission ; grossissement 120.000).

### Exemple 2

On répète le mode opératoire de l'exemple 1 à l'aide des quantités suivantes des mêmes réactifs :

### 1ère étape

| | |
|---|---|
| - Résine A 123 | 6 g |
| - GAFAC RE 610 | 4 g |
| - Styrène | 60 g |
| - Pigment magnétique | 30 g |

### 2ème étape

| | |
|---|---|
| - Peroxyde de dioctanoyle | 0,7 g |
| - Divinylbenzène | 1,5 g |
| - Phase organique de la 1ère étape | 40 g |
| - Eau à pH 10 | 200 g |

### 3ème étape

Les conditions de polymérisation sont identiques ; 50 % du poids des microsphères formées sont magnétisables.

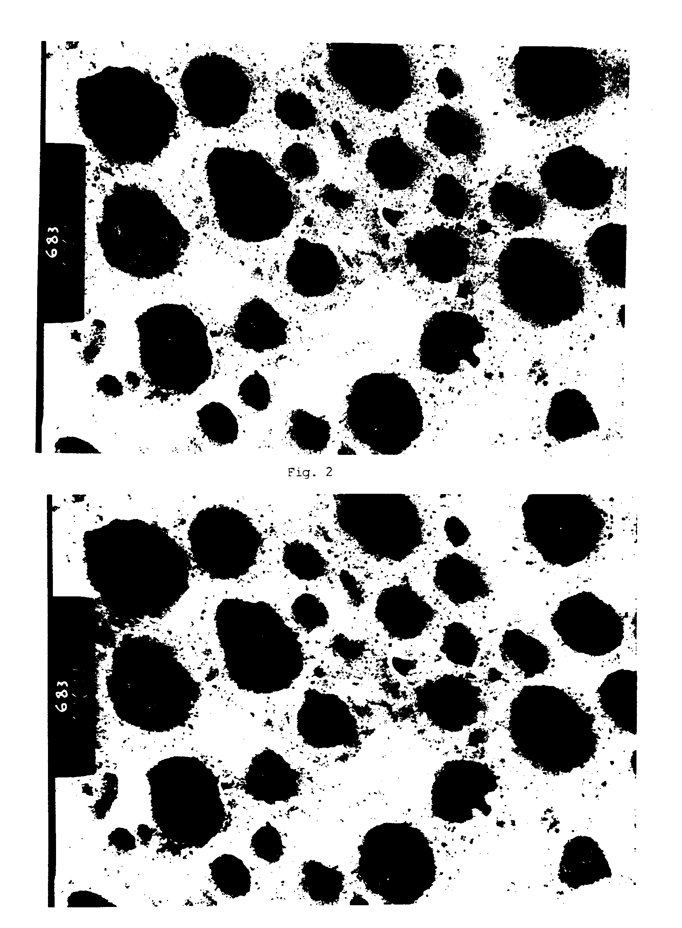

Les microsphères magnétisables présentent un diamètre moyen de l'ordre de 0,6 »m et renferment 43 % de leur poids de particules de ferrite formant le coeur des microsphères, comme le montre la photo de la figure n° 2 (grossissement 44.000).

## Revendications (Revendications pour l'(les) Etat(s) contractant(s) suivant(s): AT, BE, CH, DE, DK, FR, GB, GR, IT, LI, LU, NL, SE)

1. Microsphères composites magnétisables de polymère vinylaromatique réticulé hydrophobe caractérisées en ce que :
- elles présentent un diamètre de 0,05 à 10 »m
- elles comprennent :
. un coeur essentiellement constitué de particules magnétisables présentant un diamètre inférieur à 300 10⁻⁴ »m
. et une écorce constituée d'un copolymère réticulé hydrophobe dérivé d'au moins un monomètre vinylaromatique hydrophobe et d'au moins un polymère émulsifiant polyéthyléniquement insaturé soluble dans ledit ou lesdits monomère(s) vinylaromatique(s) et susceptible de réticuler avec ledit ou lesdits monomère(s) vinylaromatique(s).

2. Microsphères selon la revendication 1 caractérisées en ce que le coeur en particules magnétisables représente de 0,5 à 70 % du poids desdites microsphères magnétisables.

3. Microsphéres selon la revendication 1 ou 2 caractérisées en ce que les particules magnétisables sont de la magnétite ou de l'hematite.

4. Microsphères selon l'une quelconque des revendications 1 à 3 caractérisées en ce que le polymère émulsifiant réticulant est un polyester d'alkylène glycol et de diacide insaturé aliphatique et/ou aromatique de masse moléculaire en poids de 500 à 10.000.

5. Microsphéres selon l'une quelconque des revendications précédentes caractérisées en ce que le polymère émulsifiant réticulant représente de 3 à 15 % du poids de monomère vinylaromatique hydrophobe.

6. Dispersion aqueuse caractérisée en ce qu'elle contient 10 à 70 % en poids de microsphères selon l'une des revendications précédentes.

7. Procédé de préparation de microsphères magnétisables se présentant telles quelles ou en dispersion dans l'eau caractérisé en ce qu'il consiste :
- à disperser des particules magnétisables présentant un diamètre inférieur à 300 10⁻⁴ »m, revêtues d'une couche monomoléculaire d'un agent dispersant non hydrosoluble dans un mélange constitué d'au moins un monomère vinylaromatique hydrophobe, d'au moins un polymère émulsifiant polyéthylèniquement insaturé soluble dans ledit ou lesdits monomère(s) vinylaromatique(s) et susceptible de réticuler avec ledit ou lesdits monomère(s) vinylaromatique(s) et éventuellement d'un agent dispersant complémentaire.
- à préémulsionner le fluide magnétique ainsi obtenu dans de l'eau, après introduction préalable dans ledit fluide d'un amorceur organosoluble de polymérisation de façon à obtenir une émulsion de gouttelettes de fluide magnétique de taille de 0,03 à 10 »m.
- à copolymériser les composés éthylèniquement insaturés présents dans le milieu
- à séparer les microsphères magnétisables formées des microsphères non magnétisables
- et éventuellement à redisperser dans l'eau les microsphères magnétisables.

8. Procédé selon la revendication 7/ caractérisé en ce que les particules magnétisables représentent de 0,5 à 60 % environ du poids de composés éthyléniquement insaturés copolymérisables.

9. Procédé selon la revendication 7/ ou 8/ caractérisé en ce que les particules magnétisables sont de la magnétite ou de l'hématite.

10. Procédé selon l'une quelconque des revendications 7/ à 9/ caractérisé en ce que l'agent dispersant non hydrosoluble est un acide gras ou une amine grasse contenant au moins 12 atomes de carbone.

11. Procécé selon l'une quelconque des revendications précédentes caractérisé en ce que le polymère émulsifiant réticulant est un polyester d'alkylène glycol et de diacide insaturé aliphatique et/ou aromatique de masse moléculaire en poids de 500 à 10.000.

12. Procécé selon l'une quelconque des revendications précédentes caractérisé en ce que le polymère émulsifiant réticulant représente de 3 à 15 % du poids de monomère vinylaromatique hydrophobe.

13. Procédé selon l'une quelconque des revendications précédentes caractérisé en ce que la quantité d'amorceur organosoluble est de 0,1 à 10 % du poids de composés éthyléniquement insaturés copolymérisables.

14. Utilisation des microsphères magnétisables faisant l'objet de l'une quelconque des revendications 1 à 6 pour immobiliser des substances biologiquement actives.

## Revendications (Revendications pour l'(les) Etat(s) contractant(s) suivant(s): ES)

1. Procédé de préparation de microsphères magnétisables se présentant telles quelles ou en dispersion dans l'eau caractérisé en ce qu'il consiste :
- à disperser des particules magnétisables présentant un diamètre inférieur à 300 10⁻⁴ »m, revêtues d'une couche monomoléculaire d'un agent dispersant non hydrosoluble dans un mélange constitué d'au moins un monomère vinylaromatique hydrophobe, d'au moins un polymère émulsifiant polyéthylèniquement insaturé soluble dans ledit ou lesdits monomère(s) vinylaromatique(s) et susceptible de réticuler avec ledit ou lesdits monomère(s) vinylaromatique(s) et éventuellement d'un agent dispersant complémentaire.
- à préémulsionner le fluide magnétique ainsi obtenu dans de l'eau, après introduction préalable dans ledit fluide d'un amorceur organosoluble de polymérisation de façon à obtenir une émulsion de gouttelettes de fluide magnétique de taille de 0.03 à 10 »m.
- à copolymériser les composés éthylèniquement insaturés présents dans le milieu
- à séparer les microsphères magnétisables formées des microsphères non magnétisables
- et éventuellement à redisperser dans l'eau les microsphères magnétisables.

2. Procédé selon la revendication 1 / caractérisé en ce que les particules magnétisables représentent de 0,5 à 60 % du poids de composés éthyléniquement insaturés copolymérisables.

3. Procédé selon la revendication 1/ ou 2/ caractérisé en ce que les particules magnétisables sont de la magnétite ou de l'hématite.

4. Procédé selon l'une quelconque des revendications 1/ à 3 / caractérisé en ce que l'agent dispersant non hydrosoluble est un acide gras ou une amine grasse contenant au moins 12 atomes de carbone.

5. Procécé selon l'une quelconque des revendications précédentes caractérisé en ce que le polymère émulsifiant réticulant est un polyester d'alkylène glycol et de diacide insaturé aliphatique et/ou aromatique de masse moléculaire en poids de 500 à 10.000.

6. Procécé selon l'une quelconque des revendications précédentes caractérisé en ce que le polymère émulsifiant réticulant représente de 3 à 15 % du poids de monomère vinylaromatique hydrophobe.

7. Procédé selon l'une quelconque des revendications précédentes caractérisé en ce que la quantité d'amorceur organosoluble est de 0,1 à 10 % du poids de composés éthyléniquement insaturés copolymérisables.

8. Utilisation des microsphères magnétisables obtenues selon le procédé faisant l'objet de l'une quelconque des revendications 1 à 7 pour immobiliser des substances biologiquement actives.

## Claims (Claims for the following Contracting State(s): AT, BE, CH, DE, DK, FR, GB, GR, IT, LI, LU, NL, SE)

1. Magnetizable composite microspheres of hydrophobic crosslinked vinylaromatic polymer, characterized in that:
- they have a diameter of 0.05 to 10 »m
- they include:
a core consisting essentially of magnetizable particles which have a diameter smaller than 300 10⁻⁴ »m
and a shell consisting of a hydrophobic crosslinked copolymer derived from at least one hydrophobic vinylaromatic monometre [sic] and from at least one polyethylenically unsaturated emulsifying polymer soluble in the said vinylaromatic monomer(s) and capable of crosslinking with the said vinylaromatic monomer(s).

2. Microspheres according to Claim 1, characterized in that the core made of magnetizable particles represents from 0.5 to 70 % of the weight of the said magnetizable microspheres.

3. Microspheres according to Claim 1 or 2, characterized in that the magnetizable particles are magnetite or haematite.

4. Microspheres according to any one of Claims 1 to 3, characterized in that the crosslinking emulsifying polymer is a polyester of alkylene glycol and of unsaturated aliphatic and/or aromatic diacid, of weight molecular mass of 500 to 10,000.

5. Microspheres according to any one of the preceding claims, characterized in that the crosslinking emulsifying polymer represents from 3 to 15 % of the weight of hydrophobic vinylaromatic monomer.

6. Aqueous dispersion characterized in that it contains 10 to 70 % by weight of microspheres according to one of the preceding claims.

7. Process for the preparation of magnetizable microspheres which are as such or in dispersion in water, characterized in that it consists:
- in dispersing magnetizable particles which have a diameter of less than 300 10⁻⁴ »m, coated with a monomolecular layer of a non-water-soluble dispersing agent in a mixture consisting of at least one hydrophobic vinylaromatic monomer, of at least one polyethylenically unsaturated emulsifying polymer soluble in the said vinylaromatic monomer(s) and capable of crosslinking with the said vinylaromatic monomer(s) and optionally of a complementary dispersing agent.
- in preemulsifying the magnetic fluid thus obtained in water, after previous introduction into the said fluid of an organosoluble polymerization initiator so as to obtain an emulsion of droplets of magnetic fluid from 0.03 to 10 »m in size.
- in copolymerizing the ethylenically unsaturated compounds present in the mixture
- in separating the magnetizable microspheres formed from the nonmagnetizable microspheres
- and optionally in redispersing the magnetizable microspheres in water.

8. Process according to Claim 7, characterized in that the magnetizable particles represent from 0.5 to 60 % of the weight of copolymerizable ethylenically unsaturated compounds.

9. Process according to Claim 7 or 8, characterized in that the magnetizable particles are magnetite or haematite.

10. Process according to any one of Claims 7 to 9, characterized in that the non-water-soluble dispersing agent is a fatty acid or a fatty amine containing at least 12 carbon atoms.

11. Process according to any one of the preceding claims, characterized in that the crosslinking emulsifying polymer is a polyester of alkylene glycol and of unsaturated aliphatic and/or aromatic diacid of weight molecular mass of 500 to 10,000.

12. Process according to any one of the preceding claims, characterized in that the crosslinking emulsifying polymer represents from 3 to 15 % of the weight of hydrophobic vinylaromatic monomer.

13. Process according to any one of the preceding claims, characterized in that the quantity of organosoluble initiator is 0.1 to 10 % of the weight of copolymerizable ethylenically unsaturated compounds.

14. Use of the magnetizable microspheres forming the subject of any one of Claims 1 to 6 for immobilizing biologically active substances.

## Claims (Claims for the following Contracting State(s): ES)

1. Process for the preparation of magnetizable microspheres which are as such or in dispersion in water, characterized in that it consists:
- in dispersing magnetizable particles which have a diameter of less than 300 10⁻⁴ »m, coated with a monomolecular layer of a non-water-soluble dispersing agent in a mixture consisting of at least one hydrophobic vinylaromatic monomer, of at least one polyethylenically unsaturated emulsifying polymer soluble in the said vinylaromatic monomer(s) and capable of crosslinking with the said vinylaromatic monomer(s) and optionally of a complementary dispersing agent.
- in preemulsifying the magnetic fluid thus obtained in water, after previous introduction into the said fluid of an organosoluble polymerization initiator so as to obtain an emulsion of droplets of magnetic fluid from 0.03 to 10 »m in size.
- in copolymerizing the ethylenically unsaturated compounds present in the mixture
- in separating the magnetizable microspheres formed from the nonmagnetizable microspheres
- and optionally in redispersing the magnetizable microspheres in water.

2. Process according to Claim 1, characterized in that the magnetizable particles represent from 0.5 to 60 % of the weight of copolymerizable ethylenically unsaturated compounds.

3. Process according to Claim 1 or 2, characterized in that the magnetizable particles are magnetite or haematite.

4. Process according to any one of Claims 1 to 3, characterized in that the non-water-soluble dispersing agent is a fatty acid or a fatty amine containing at least 12 carbon atoms.

5. Process according to any one of the preceding claims, characterized in that the crosslinking emulsifying polymer is a polyester of alkylene glycol and of unsaturated aliphatic and/or aromatic diacid of weight molecular mass of 500 to 10,000.

6. Process according to any one of the preceding claims, characterized in that the crosslinking emulsifying polymer represents from 3 to 15 % of the weight of hydrophobic vinylaromatic monomer.

7. Process according to any one of the preceding claims, characterized in that the quantity of organosoluble initiator is 0.1 to 10 % of the weight of copolymerizable ethylenically unsaturated compounds.

8. Use of the magnetizable microspheres forming the subject of and obtained according to the process of any one of Claims 1 to 7 for immobilizing biologically active substances.

## Patentansprüche (Patentansprüche für folgende(n) Vertragsstaat(en): AT, BE, CH, DE, DK, FR, GB, GR, IT, LI, LU, NL, SE)

1. Magnetisierbare Mikrokugel-Komposite aus hydrophobem vernetzten vinylaromatischen Polymer, dadurch gekennzeichnet, daß
- sie einen Durchmesser von 0,05 bis 10 »m aufweisen,
- sie umfassen:
· einen Kern, der im wesentlichen aus magnetisierbaren Teilchen mit einem Durchmesser von unter 300·10⁻⁴ »m besteht,
· und eine Rinde, die aus einem hydrophoben vernetzten Copolymer besteht, das von mindestens einem hydrophoben vinylaromatischen Monomer und mindestens einem polyethylenisch ungesättigten, emulgierenden Polymer abgeleitet ist, das in dem oder den genannten vinylaromatischen Monomer(en) löslich und fähig ist, mit dem oder den genannten vinylaromatischen Monomer(en) zu vernetzen.

2. Mikrokugeln nach Anspruch 1, dadurch gekennzeichnet, daß der Kern aus magnetisierbaren Teilchen 0,5 bis 70 Gew.-% der genannten magnetisierbaren Mikrokugeln darstellt.

3. Mikrokugeln nach Anspruch 1 oder 2, dadurch gekennzeichnet, daß die magnetisierbaren Teilchen Magnetit oder Hämatit sind.

4. Mikrokugeln nach irgendeinem der Ansprüche 1 bis 3, dadurch gekennzeichnet, daß das vernetzende, emulgierende Polymer ein Polyester von Alkylenglycol und einer ungesättigten, aliphatischen und/oder aromatischen Disäure mit einer Molekularmasse in Gewicht von 500 bis 10.000 ist.

5. Mikrokugeln nach irgendeinem der vorstehenden Ansprüche, dadurch gekennzeichnet, daß das vernetzende, emulgierende Polymer 3 bis 15 Gew.-% des hydrophoben vinylaromatischen Monomers darstellt.

6. Wäßrige Dispersion, dadurch gekennzeichnet, daß sie 10 bis 70 Gew.-% der Mikrokugeln nach einem der vorstehenden Ansprüche enthält.

7. Verfahren zur Herstellung von magnetisierbaren Mikrokugeln, die so, wie sie sind oder als wäßrige Dispersion vorliegen, dadurch gekennzeichnet, daß es darin besteht:
- magnetisierbare Teilchen, die einen unteren Durchmesser von 300·10⁻⁴ »m aufweisen und mit einer monomolekularen Schicht eines nicht wasserlöslichen Dispergierungsmittels überzogen sind, in einer Mischung zu dispergieren, die mindestens aus einem hydrophoben vinylaromatischen Monomer und mindestens einem polyethylenisch ungesättigten, emulgierenden Polymer, das in dem oder den genannten vinylaromatischen Monomer(en) löslich und fähig ist, mit dem oder den genannten vinylaromatischen Monomer(en) zu vernetzen, und gegebenenfalls einem zusätzlichen Dispergierungsmittel besteht;
- das auf diese Weise erhaltene magnetische Fluid nach vorherigem Eintragen eines organolöslichen Polymerisations-Initiators in Wasser vorzuemulgieren, so daß eine Emulsion von Tröpfchen des magnetischen Fluids mit einer Größe von 0,03 bis 10 »m erhalten wird;
- die in dem Milieu vorliegenden ethylenisch ungesättigten Verbindungen zu copolymerisieren;
- die gebildeten magnetisierbaren Mikrokugeln von den nicht magnetisierbaren Mikrokugeln abzutrennen; und
- gegebenenfalls die magnetisierbaren Mikrokugeln in Wasser zurückzudispergieren.

8. Verfahren nach Anspruch 7, dadurch gekennzeichnet, daß die magnetisierbaren Teilchen 0,5 bis 60 Gew.-% der copolymerisierbaren, ethylenisch ungesättigten Verbindungen darstellen.

9. Verfahren nach Anspruch 7 oder 8, dadurch gekennzeichnet, daß die magnetisierbaren Teilchen Magnetit oder Hämatit sind.

10. Verfahren nach irgendeinem der Ansprüche 7 bis 9, dadurch gekennzeichnet, daß das nicht wasserlösliche Dispergierungsmittel eine Fettsäure oder ein Fettamin mit mindestens 12 Kohlenstoffatomen ist.

11. Verfahren nach irgendeinem der vorstehenden Ansprüche, dadurch gekennzeichnet, daß das vernetzende, emulgierende Polymer ein Polyester von Alkylenglycol und einer ungesättigten, aliphatischen und/oder aromatischen Disäure mit einer Molekularmasse in Gewicht von 500 bis 10.000 ist.

12. Verfahren nach irgendeinem der vorstehenden Ansprüche, dadurch gekennzeichnet, daß das vernetzende, emulgierende Polymer 3 bis 15 Gew.-% des hydrophoben vinylaromatischen Monomers darstellt.

13. Verfahren nach irgendeinem der vorstehenden Ansprüche, dadurch gekennzeichnet, daß die Menge des organolöslichen Initiators 0,1 bis 10 Gew.-% der copolymerisierbaren, ethylenisch ungesättigten Verbindungen beträgt.

14. Verwendung der magnetisierbaren Mikrokugeln nach einem der Ansprüche 1 bis 6 zur Immobilisierung von biologisch aktiven Substanzen.

## Patentansprüche (Patentansprüche für folgende(n) Vertragsstaat(en): ES)

1. Verfahren zur Herstellung von magnetisierbaren Mikrokugeln, die so, wie sie sind oder als wäßrige Dispersion vorliegen, dadurch gekennzeichnet, daß es darin besteht:
- magnetisierbare Teilchen, die einen unteren Durchmesser von 300·10⁻⁴ »m aufweisen und mit einer monomolekularen Schicht eines nicht wasserlöslichen Dispergierungsmittels überzogen sind, in einer Mischung zu dispergieren, die mindestens aus einem hydrophoben vinylaromatischen Monomer und mindestens einem polyethylenisch ungesättigten, emulgierenden Polymer, das in dem oder den genannten vinylaromatischen Monomer(en) löslich und fähig ist, mit dem oder den genannten vinylaromatischen Monomer(en) zu vernetzen, und gegebenenfalls einem zusätzlichen Dispergierungsmittel besteht;
- das auf diese Weise erhaltene magnetische Fluid nach vorherigem Eintragen eines organolöslichen Polymerisations-Initiators in Wasser vorzuemulgieren, so daß eine Emulsion von Tröpfchen des magnetischen Fluids mit einer Größe von 0,03 bis 10 »m erhalten wird;
- die in dem Milieu vorliegenden ethylenisch ungesättigten Verbindungen zu copolymerisieren;
- die gebildeten magnetisierbaren Mikrokugeln von den nicht magnetisierbaren Mikrokugeln abzutrennen; und
- gegebenenfalls die magnetisierbaren Mikrokugeln in Wasser zurückzudispergieren.

2. Verfahren nach Anspruch 1, dadurch gekennzeichnet, daß die magnetisierbaren Teilchen 0,5 bis 60 Gew.-% der copolymerisierbaren, ethylenisch ungesättigten Verbindungen darstellen.

3. Verfahren nach Anspruch 1 oder 2, dadurch gekennzeichnet, daß die magnetisierbaren Teilchen Magnetit oder Hämatit sind.

4. Verfahren nach irgendeinem der Ansprüche 1 bis 3, dadurch gekennzeichnet, daß das nicht wasserlösliche Dispergierungsmittel eine Fettsäure oder ein Fettamin mit mindestens 12 Kohlenstoffatomen ist.

5. Verfahren nach irgendeinem der vorstehenden Ansprüche, dadurch gekennzeichnet, daß das vernetzende, emulgierende Polymer ein Polyester von Alkylenglycol und einer ungesättigten, aliphatischen und/oder aromatischen Disäure mit einer Molekularmasse in Gewicht von 500 bis 10.000 ist.

6. Verfahren nach irgendeinem der vorstehenden Ansprüche, dadurch gekennzeichnet, daß das vernetzende, emulgierende Polymer 3 bis 15 Gew.-% des hydrophoben vinylaromatischen Monomers darstellt.

7. Verfahren nach irgendeinem der vorstehenden Ansprüche, dadurch gekennzeichnet, daß die Menge des organolöslichen Initiators 0,1 bis 10 Gew.-% der copolymerisierbaren, ethylenisch ungesättigten Verbindungen beträgt.

8. Verwendung der magnetisierbaren Mikrokugeln, erhalten nach dem Verfahren gemäß einem der Ansprüche 1 bis 7, zur Immobilisierung von biologisch aktiven Substanzen.
